# EUROPÄISCHE PATENTSCHRIFT

(11) **EP 4 358 922 B1**
(45) Veröffentlichungstag und Bekanntmachung des Hinweises auf die Patenterteilung: **12.08.2026**
(21) Anmeldenummer: 22726772.1
(22) Anmeldetag: 10.05.2022
(51) Int. Cl.: A61K 8/02, A61K 8/44, A61K 8/46, A61K 8/73, A61Q 5/02, A61Q 19/10

(54) **FESTE UND PULVERFÖRMIGE FORMULIERUNGEN**
SOLID AND POWDERY FORMULATIONS
FORMULATIONS SOLIDES ET PULVÉRULENTES

(30) Priorität: 22.06.2021 DE 102021206380
(43) Veröffentlichungstag der Anmeldung: 01.05.2024
(73) Patentinhaber: Beiersdorf AG, 22529 Hamburg (DE)
(72) Erfinder: KOOP, Eefje, 22850 Norderstedt (DE); SCHLÜSCHE, Wiebke, 25469 Halstenbek (DE); RATSCHOW, Cecile, 22761 Hamburg (DE)
(74) Vertreter: Beiersdorf AG
(86) Internationale Anmeldenummer: PCT/EP2022/062570
(87) Internationale Veröffentlichungsnummer: WO 2022/268396

(56) Entgegenhaltungen:
- WO-A1-2015/158638
- DE-A1- 10 330 247
- DE-A1- 102019 219 714
- FR-A1- 3 104 025
- KR-A- 20090 073 621
- US-A- 5 900 230
- US-A1- 2016 235 634

## Beschreibung

Kosmetische Produkte dienen im Allgemeinen nicht nur dazu schön und attraktiv auszusehen, sondern sie tragen mit ihrer Wirkung entscheidend zu einem gesteigerten Selbstwertgefühl und zum Wohlbefinden der Menschen bei. Dementsprechend werden die verschiedensten kosmetischen Produkte zur täglichen Reinigung und Pflege der menschlichen Haut eingesetzt.

Herkömmliche Reinigungsformulierungen werden meist in Form einer flüssigen, wässrigen Formulierung verkauft. Analysiert man die Inhaltsstoffe einer solchen Formulierung, kann man feststellen, dass die Formulierung eine signifikante Menge an Wasser enthält. Typische Beispiele für Duschgels, die 20 Gew.-% bis 95 Gew.-% Wasser enthalten, sind in WO1994010975A1, WO1993021900A1 und WO2002005758A2 offenbart.

Dementsprechend werden erhebliche Mengen an Wasser vom Lieferanten zu den Geschäften und weiter zum Kunden transportiert, um den Kunden mit der Reinigungsformel seiner Wahl zu versorgen. In Anbetracht der Tatsache, dass die meisten Häuser der Kunden an die öffentliche Wasserversorgung angeschlossen sind, erscheint es aus ökologischer Sicht nicht sinnvoll, Wasser an einen Ort zu versenden, der grundsätzlich unbegrenzten Zugang zu Wasser hat. Durch den Verzicht auf Wasser in den Rezepturen und den Versand eines Konzentrats in flüssiger oder fester Form können also Transportkosten und transportbedingte Emissionen von Treibhausgasen reduziert werden.

Neuartige Konzepte beinhalten z. B. den Versand von Konzentraten und eine Vermischung mit Wasser am Point of Sale. Andere Ansätze verwenden feste Reinigungskompositionen in Form eines Riegels. Die letztgenannten Formulierungen werden vom Kunden wie ein Seifenstück aufgetragen, z. B. durch Reiben des Stücks über den Körper. Die Verwendung eines festen Riegels zum Auftragen von Waschwirkstoffen auf den menschlichen Körper ist jedoch oft nicht wünschenswert für Kunden, die gewohnt sind, Reinigungsformulierungen in flüssiger Form aufzutragen.

Folglich können umweltfreundliche kosmetische Reinigungszubereitungen ebenfalls in Form von Konzentraten an den Verbraucher geliefert werden. Dieser löst die Konzentrate in vorgegebenen Anteilen mit Wasser, so dass eine kosmetische Reinigungszubereitung entsteht. Durch diese Anwendungsform wird Wasser in der Produktion der Konzentrate gespart. Dieses führt durch Gewichtsminimierung zu einer geringeren Umweltbelastung, da der Verbraucher Wasser zumeist zuhause zufügen kann.

Das Konzentrat kann dabei in flüssiger oder fester Form vorliegen. Bei der festen Form muss jedoch zwischen pulverförmigen Zubereitungen, welche rieselfähig sind, und zwischen Zubereitungen, welche als einzelnes, festes Stück vorliegen, unterschieden werden. Das feste Stück kann durch Verpreussung einer pulverförmigen Zubereitung erhalten werden oder durch aufschmelzen und abkühlen der Zubereitung zu einem verfestigten zusammenhängenden Gebilde erhalten werden.

Die vorliegende Anmeldung betrifft vorwiegend Konzentrate in fester Form. Ziel ist es, dass der Verbraucher nach dem Vermischen mit Wasser eine Reinigungszubereitung erhält, die mittels eines Pumpschäumers aufgeschäumt werden kann und bei Anwendung zur Reinigung die gewohnte Waschwirkung und Cremigkeit vermittelt. Cremigkeit setzt voraus, dass Polymere in die Zubereitung eingearbeitet sind, welche die Viskosität gegenüber Wasser leicht erhöhen. Ein bekanntes Biopolymer oftmals in Reinigungszubereitungen enthalten ist Xanthan Gum.

Problematische ist jedoch, dass pulverförmige kosmetische Reinigungszusammensetzung mit Xanthan Gum nach dem Lösen in Wasser durch den Verbraucher oftmals Instabilitäten insbesondere mit unter Normalbedingungen pulverförmigen, festen Tensiden zeigen und Ausfällungen auftreten oder es zu einer ungleichmäßigen Verdickung der Zusammensetzung kommt. Dieser Nachteil sollte durch die vorliegende Erfindung adressiert werden.

Im Stand der Technik sind Konzentrate aus den Dokumenten DE102019219714 A1, FR 3104025 A1, KR 20090073621 A und DE 10330247 A1 bekannt. Jedoch konnte keines dieser Dokumente einen Hinweis auf die vorliegende Erfindung liefern.

Überraschend wurde nun gefunden, dass die Nachteile des Standes der Technik durch die vorliegende Erfindung beseitigt bzw. gelindert werden konnten.

Gegenstand der vorliegenden Erfindung ist ein kosmetische Reinigungskonzentrat in fester Form aufweisend, bezogen auf das Gesamtgewicht des Konzentrats,
a) ein oder mehrere unter Normalbedingungen feste Tenside und
b) Caesalpinia Spinosa Gum und/oder Cellulose Gum;
dadurch gekennzeichnet, dass das Konzentrat weniger als 7% unter Normalbedingungen flüssige Inhaltstoffe enthält und Sodium Benzoat enthalten ist.

Sollten nachfolgend Gewichtsprozentangaben (Gew.-%) ohne Bezugnahme auf eine bestimmte Zusammensetzung oder spezifische Mischung angegeben werden, so beziehen sich diese Angaben immer auf das Gesamtgewicht des Reinigungskonzentrats. Sollten nachfolgend Verhältnisse von Komponenten/Substanzen/Stoffgruppen offenbart werden, so beziehen sich diese Verhältnisse auf Gewichtsverhältnisse der genannten Komponenten/ Substanzen/Stoffgruppen.

Werden nachfolgend Gewichtsprozentbereiche für die Bestandteile des Reinigungskonzentrats angegeben, so umfasst die Offenbarung der vorliegenden Anmeldung ebenfalls alle Einzelwerte in Schritten von 0,1 Gew.-% innerhalb dieser Gewichtsprozentbereiche.

Die Formulierungen "erfindungsgemäß", "erfindungsgemäß vorteilhaft", "vorteilhaft im Sinne der vorliegenden Erfindung" etc. beziehen sich im Rahmen der vorliegenden Offenbarung immer sowohl auf das erfindungsgemäße Reinigungskonzentrat sowie die erfindungsgemäße Verwendung und das Verfahren.

Sofern nicht anders angegeben wurden alle Versuche unter Normalbedingungen durchgeführt. Der Begriff "Normalbedingungen" bedeutet 20°C, 1013 hPa und eine relative Luftfeuchtigkeit von 50%.

Wird der Begriff Haut verwendet, so bezieht dieser sich bevorzugt auf die menschliche Haut.

Alle nachfolgend genannten Substanzen werden anhand Ihre INCI Deklaration oder mittels eindeutigem chemischem Namen benannt.

Unter Emulgatoren werden alle Substanzen verstanden, welche im International Cosmetic Ingredient Dictionary and Handbook, Thirteenth Edition 2010, (ISBN 1-882621-47-6) unter der Bezeichnung "emulsifying agent" geführt werden. Unter Tensiden werden alle Substanzen verstanden, welche im International Cosmetic Ingredient Dictionary and Handbook, Thirteenth Edition 2010, (ISBN 1-882621-47-6) unter der Bezeichnung " surfactant " geführt werden.

Werden in dieser Offenbarung Viskositätswerte angegeben, so beziehen sich alle Werte auf eine Messung bei 25°C in einer 150 ml Weithalsflasche (VWR Nr.: 807-001) mittels Rheomat R 123 von der Firma proRheo. Der Rheomat R 123 der Firma proRheo GmbH ist ein Rotationsviskosimeter, d. h. ein Messkörper rotiert in der zu vermessenden Substanz. Es wird die Kraft gemessen, die benötigt wird, um den Messkörper in der Probe mit einer vorgegebenen Drehzahl rotieren zu lassen. Aus diesem Drehmoment, der Drehzahl des Messkörpers und den geometrischen Abmessungen des verwendeten Messsystems wird die Viskosität berechnet. Als Messkörper wird der Messkörper Nr.1 (Artikelnr. 200 0191), geeignet für einen Viskositätsbereich bis 10.000 [mPa·s], Drehzahl Bereich 62,5 min-1, verwendet.

Erfindungsgemäß umfasst das kosmetische Reinigungskonzentrat mindestens ein unter Normalbedingungen festes Tensid. Vorteilhafte Tenside sind gewählt aus der Gruppe Sodium Myristoyl Glutamate, Sodium Lauroyl Glutamate, Sodium Stearoyl Glutamate, Sodium Cocoyl Glutamate, Sodium Coco Sulfate, Disodium Lauryl Sulfosuccinate und Sodium Methyl Cocoyl Taurate. Es ist insbesondere vorteilhaft, wenn Disodium Lauryl Sulfosuccinate und/oder Sodium Coco Sulfate im Reinigungskonzentrat enthalten sind.

Es ist vorteilhaft im Sinne der vorliegenden Erfindung, wenn der Anteil von unter Normalbedingungen festen Tenside von 0,1 bis 50 Gew.-%, bevorzugt von 15 bis 45 Gew.-% und insbesondere bevorzugt von 25 bis 44 Gew.-%, bezogen auf das Gesamtgewicht des Reinigungskonzentrats, beträgt.

Es ist vorteilhaft im Sinne der vorliegenden Erfindung, wenn der Anteil von unter Normalbedingungen festen Tenside gewählt aus der Gruppe Sodium Myristoyl Glutamate, Sodium Lauroyl Glutamate, Sodium Stearoyl Glutamate, Sodium Cocoyl Glutamate, Sodium Coco Sulfate, Disodium Lauryl Sulfosuccinate und Sodium Methyl Cocoyl Taurate von 0,1 bis 50 Gew.-%, bevorzugt von 15 bis 45 Gew.-% und insbesondere bevorzugt von 25 bis 44 Gew.-%, bezogen auf das Gesamtgewicht des Reinigungskonzentrats, beträgt.

Es ist insbesondere vorteilhaft im Sinne der Erfindung, wenn der Anteil von Disodium Lauryl Sulfosuccinate und/oder Sodium Coco Sulfate von 0,1 bis 50 Gew.-%, bevorzugt von 15 bis 45 Gew.-%, bevorzugt von 25 bis 44 Gew.-% und insbesondere bevorzugt von 30 bis 43 Gew.-%, bezogen auf das Gesamtgewicht des Reinigungskonzentrats, beträgt.

Disodium Lauryl Sulfosuccinate ist vorteilhaft in einem Anteil von 8 bis 30 Gew.-%, bevorzugt 10 bis 25 Gew.-% und insbesondere bevorzugt von 12 bis 20 Gew.-%, bezogen auf das Gesamtgewicht des Reinigungskonzentrats, enthalten.

Sodium Coco Sulfate ist vorteilhaft in einem Anteil von 12 bis 34 Gew.-%, bevorzugt 14 bis 30 Gew.-% und insbesondere bevorzugt von 18 bis 25 Gew.-%, bezogen auf das Gesamtgewicht des Reinigungskonzentrats, enthalten.

Weiterhin ist es vorteilhaft, wenn Disodium Lauryl Sulfosuccinate und Sodium Coco Sulfate enthalten sind und weiterhin vorteilhaft das Gewichtsverhältnis von Disodium Lauryl Sulfosuccinate zu Sodium Coco Sulfate von 1,5:1 bis 1:2, bevorzugt 1:1 bis 1:1,5 und insbesondere bevorzugt 1:1,1 bis 1:1,4 beträgt.

Weiterhin ist es erfindungsgemäß, dass Caesalpinia Spinosa Gum und/oder Cellulose Gum enthalten sind. Vorteilhaft beträgt der Gesamtanteil von Caesalpinia Spinosa Gum und/oder Cellulose Gum von 0,1 bis 30 Gew.-%, bevorzugt von 0,2 bis 25 Gew.-%, weiter bevorzugt von 0,3 bis 20 Gew.-% und insbesondere bevorzugt 10 bis 20 Gew.-%, bezogen auf das Gesamtgewicht des Konzentrats.

Sofern Caesalpinia Spinosa Gum enthalten ist, beträgt der Anteil von Caesalpinia Spinosa Gum vorteilhaft von 0,1 bis 30 Gew.-%, bevorzugt von 0,2 bis 25 Gew.-%, weiter bevorzugt von 0,25 bis 20 Gew.-% und insbesondere bevorzugt 0,3 bis 15 Gew.-%, bezogen auf das Gesamtgewicht des Konzentrats.

Sofern Cellulose Gum enthalten ist, beträgt der Anteil von Cellulose Gum vorteilhaft von 0,1 bis 30 Gew.-%, bevorzugt von 0,2 bis 25 Gew.-%, weiter bevorzugt von 0,25 bis 20 Gew.-% und insbesondere bevorzugt 0,3 bis 15 Gew.-%, bezogen auf das Gesamtgewicht des Konzentrats.

Während beide Polymere eine klumpenfreie/ausfällungsfreie Zubereitung bei Lösen des Konzentrats in Wasser ermöglichen, wurde überraschend festgestellt, dass durch die ausschließliche Verwendung von Caesalpinia Spinosa Gum eine besonders gleichmäßigere Verdickung erzielt wird.

Entsprechend sind Ausführungsformen enthaltend Caesalpinia Spinosa Gum bevorzugt, wobei vorteilhaft weitere Polymere in Anteilen von weniger als 10 Gew.-% enthalten sind. Es ist insbesondere vorteilhaft, wenn nur Caesalpinia Spinosa Gum enthalten ist.

Weiterhin ist es bevorzugt, wenn das Konzentrat frei von Xanthan Gum, Sphingomonas Ferment Extract, Dehydroxanthan Gum und/oder Hydroxypropyl Guar ist.

Weiterhin ist es bevorzugt, wenn das Konzentrat frei von Polymeren, welche aus einer Homo- oder Copolymerisation mit Vinylpyrrolidon, Acryl- und/oder Methacrylsäure erhalten werden. Derartige Polymere entsprechend nicht mehr den heutigen ökologischen Ansprüchen, da deren biologische Abbaubarkeit nicht abschließend geklärt ist.

Frei von Sinne der vorliegenden Erfindung bedeutet, dass der Gesamtanteil der genannten Substanzen weniger als 0,05 Gew.-% und insbesondere bevorzugt 0 Gew-% beträgt, wobei sich die Angaben auf das Gesamtgewicht des Konzentrats beziehen.

Weiterhin hat sich überraschend gezeigt, dass der Zusatz von Maltodextrin die Cremigkeit eines aus dem Konzentrat hergestellten Reinigungsprodukts nach dem Aufschäumen mit einem Pumpschäumer deutlich erhöht, ohne dabei Ausfällungen in dem Reinigungsprodukt zu erzeigen.

Entsprechend ist es vorteilhaft, wenn das Konzentrat Maltodextin umfasst. Sofern Maltodextrin enthalten ist, ist es weiterhin vorteilhaft, wenn der Anteil von Maltodextrin von 0,1 bis 20 Gew.-%, bevorzugt von 2 bis 14 Gew.-% und insbesondere bevorzugt von 3 bis 9 Gew.-%, bezogen auf das Gesamtgewicht des Konzentrats, beträgt.

Weiterhin ist es vorteilhaft, wenn das Konzentrat Magnesium Carbonate Hydroxide umfasst. Sofern Carbonate Hydroxide enthalten ist, ist es weiterhin vorteilhaft, wenn der Anteil von Carbonate Hydroxide von 0,5 bis 15 Gew.-%, bevorzugt von 1 bis 8 Gew.-% und insbesondere bevorzugt on 2 bis 6 Gew.-%, bezogen auf das Gesamtgewicht des Konzentrats, beträgt.

Weiterhin ist es erfindungsgemäß, dass das Konzentrat Sodium Benzoate umfasst. Sofern Sodium Benzoate enthalten ist, ist es weiterhin vorteilhaft, wenn der Anteil von Sodium Benzoate von 0,1 bis 30 Gew.-%, bevorzugt von 5 bis 25 Gew.-% und insbesondere bevorzugt von 10 bis 20 Gew.-%, bezogen auf das Gesamtgewicht des Konzentrats, beträgt.

Weiterhin ist es vorteilhaft, wenn das Konzentrat Hydroxyacetophenone umfasst. Sofern Hydroxyacetophenone enthalten ist, ist es weiterhin vorteilhaft, wenn der Anteil von Hydroxyacetophenone von 0,1 bis 12 Gew.-%, bevorzugt von 1 bis 10 Gew.-% und insbesondere bevorzugt von 3 bis 7 Gew.-%, bezogen auf das Gesamtgewicht des Konzentrats, beträgt.

Weiterhin ist es vorteilhaft, wenn das Konzentrat Citric Acid umfasst. Sofern Citric Acid enthalten ist, ist es weiterhin vorteilhaft, wenn der Anteil von Citric Acid von 0,1 bis 30 Gew.-%, bevorzugt von 5 bis 25 Gew.-% und insbesondere bevorzugt von 10 bis 23 Gew.-%, bezogen auf das Gesamtgewicht des Konzentrats, beträgt.

Weiterhin ist es vorteilhaft, wenn das Konzentrat Sodium Citrate umfasst. Sofern Sodium Citrate enthalten ist, ist es weiterhin vorteilhaft, wenn der Anteil von Sodium Citrate von 0,1 bis 10 Gew.-%, bevorzugt von 1 bis 5 Gew.-% und insbesondere bevorzugt von 2 bis 4 Gew.-%, bezogen auf das Gesamtgewicht des Konzentrats, beträgt.

Weiterhin ist es vorteilhaft, wenn das Konzentrat DMDM Hydantoin, Chlorphenesin, lodpropylbutylcarbamate und/oder Hexamidine Diisethionate umfasst.

Sofern DMDM Hydantoin enthalten ist, ist es vorteilhaft, wenn der Anteil von DMDM Hydantoin von 0,5 bis 12 Gew.-%, bezogen auf das Gesamtgewicht des Konzentrats, beträgt.

Sofern Chlorphenesin enthalten ist, ist es vorteilhaft, wenn der Anteil von Chlorphenesin von 0,5 bis 13 Gew.-%, bezogen auf das Gesamtgewicht des Konzentrats, beträgt.

Sofern lodpropylbutylcarbamate enthalten ist, ist es vorteilhaft, wenn der Anteil von lodpropylbutylcarbamate von 0,1 bis 13 Gew.-%, bezogen auf das Gesamtgewicht des Konzentrats, beträgt.

Sofern Hexamidine Diisethionate enthalten ist, ist es vorteilhaft, wenn der Anteil von Hexamidine Diisethionate von 0,1 bis 5 Gew.-%, bezogen auf das Gesamtgewicht des Konzentrats, beträgt.

Es ist vorteilhaft, wenn der Anteil der unter Normalbedingungen flüssigen Zusatzstoffe weniger als 6,85 Gew.-% und insbesondere weniger als 6,5 Gew.-%, bezogen auf das Gesamtgewicht des Konzentrats, beträgt.

Sofern Wasser als flüssiger Zusatzstoff in dem Konzentrat enthalten ist, beträgt der Anteil von Wasser bevorzugt weniger als 5 Gew.-% und insbesondere bevorzugt weniger als 2 Gew.-%, bezogen auf das Gesamtgewicht des Konzentrats. Diese Wasser kann insbesondere über nicht vollständig Wasserfreie Inhaltstoffe eingeschleppt werden. Entsprechend ist es insbesondere vorteilhaft, wenn dem Konzentrat kein freies Wasser zugesetzt wird.

Freies Wasser ist Wasser, welche rein, also ohne als Bestandteil anderer Inhaltstoffe zugegeben wird.

Weiterhin ist es vorteilhaft, wenn das Konzentrat ein oder mehrere Parfümstoffe umfasst. Sofern es sich um flüssige Parfümstoffe handelt, ist es bevorzugt, wenn der Anteil der flüssigen Parfümstoffe nicht mehr als 7 Gew.-% bezogen auf das Gesamtgewicht des Konzentrats, ausmacht.

Weiterhin kann das Konzentrat vorteilhaft weitere Inhaltstoffe umfassen, wie vorteilhaft Farbstoffe oder Wirkstoffe. Vorteilhaft sind und Normalbedingungen keine Wasserunlöslichen Inhaltstoffe enthalten.

Weiterhin ist das Konzentrat bevorzugt frei von Seifen. Es ist bevorzugt, wenn keine weiteren Tenside enthalten sind.

Zur Bereitstellung des kosmetischen Produkts wird das Reinigungskonzentrat mit Wasser vermischt. Vorteilhaft beträgt das Gewichtsverhältnis von Wasser und Konzentrat im Bereich von 90:1 bis 99:1, bevorzugter von 92:8 bis 98:2 und am meisten bevorzugt von 93:7 bis 97:3.

Das erfindungsgemäße Konzentrat kann in verschiedenen Ausführungsformen vorliegen. So ist es erfindungsgemäß vorteilhaft, wenn das feste Konzentrat zu einer Tablette unter Druck verpresst wird. Ein derartiges Format hat den Vorteil, dass die Tablette leicht in Wasser geben werden kann. Dabei geht kein festes Pulver durch Verschütten verloren. Folglich ist ein derartiges Format in vielerlei Hinsicht vorteilhaft für die Anwendung beim Verbraucher zuhause. Folglich liegt das Konzentrat vorteilhaft in Tablettenform vor.

Weiterhin hat sich überraschend gezeigt, dass selbst als verpresse Tabelle, die Lösungseigenschaft des erfindungsgemäßen Konzentrats besonders vorteilhaft sind. So löst sich die Tabelle besonders schnell ohne Rückstände in Wasser.

Vorteilhaft ist das Konzentrat der Erfindung dadurch gekennzeichnet, dass es als verpresse Tablette vorliegt.

Weiterhin betrifft die Erfindung auch ein kosmetisches Produkt, das eine Verpackung und das erfindungsgemäße Konzentrat umfasst, wobei die Verpackung das erfindungsgemäße Konzentrat umschließt. Dabei kann das Konzentrat als freies Pulver oder gepresste Tablette vorliegen.

In einer ersten Ausführungsform umfasst die Verpackung des Konzentrats mindestens einen Abschnitt, der aus einer wasserlöslichen Folie besteht, und mindestens einen Abschnitt, der aus einem Material besteht, das nicht wasserlöslich ist. Ein bevorzugtes Beispiel für eine solche Verpackung ist eine Kappe für eine Flasche, wobei die Kappe so gestaltet ist, dass sich der Abschnitt mit der wasserlöslichen Folie im Inneren der Flasche befindet, sobald die Kappe auf eine Flasche aufgesetzt ist. Auf diese Weise kann die Kappe auf einer mit Leitungswasser gefüllten Flasche verwendet werden. Sobald die Kappe auf der Flasche angebracht ist und Wasser in Kontakt mit dem wasserlöslichen Film kommt, wird das erfindungsgemäße Konzentrat in das Innere der Flasche freigesetzt, um eine Reinigungszusammensetzung zu bilden, die vom Kunden verwendet werden soll.

Es ist bevorzugt, wenn der Verschluss so gestaltet ist, dass er von der Flasche abgenommen werden kann, um entweder die Möglichkeit zu haben, die Reinigungszusammensetzung zur Verwendung aus der Flasche zu entnehmen, oder um den Austausch des Verschlusses zu ermöglichen, so dass die Flasche wiederverwendet werden kann. Auf diese Weise können die Transportkosten reduziert werden, da nur die Kappe an den Kunden verkauft wird, während die Flasche wiederverwendet werden kann.

Es ist weiter bevorzugt, wenn die Kappe ein Gewinde aufweist, um abnehmbar auf der Flasche befestigt zu werden. Weiterhin ist es bevorzugt, wenn die Kappe, die das Konzentrat umschließt, so ausgebildet ist, dass die Kappe eine verschließbare Öffnung aufweist, die so in der Kappe ausgerichtet ist, dass für den Fall, dass die Kappe an einer Flasche angebracht ist, der Inhalt der Flasche entnommen werden kann. Weiterhin ist die Kappe vorzugsweise dadurch gekennzeichnet, dass sie eine abnehmbare Dichtung aufweist, die so an der Kappe angebracht ist, dass sie kein Wasser mit dem wasserlöslichen Film in Berührung kommen lässt. Auf diese Weise wird vermieden, dass das Konzentrat versehentlich freigesetzt wird, wenn die Verpackung beim Transport oder zu Hause nass wird. Das Siegel kann von Hand entfernt werden, oder in bevorzugten Ausführungsformen ist das Siegel so an der Kappe angebracht, dass es automatisch bricht, sobald die Kappe auf eine passende Flasche aufgesetzt wird.

In einer anderen Ausführungsform ist die Verpackung ebenfalls eine Kappe, wie in der oben beschriebenen Ausführungsform. Diese Ausführungsform unterscheidet sich jedoch dadurch, dass das Konzentrat in der Kappe von einer Folie eingeschlossen ist, die aufreißt, wenn die Kappe auf die Flasche aufgesetzt wird, so dass das Konzentrat automatisch in das Innere der Flasche abgegeben wird.

In einer weiteren Ausführungsform ist das Konzentrat der Erfindung von der Verpackung umschlossen, wobei die Verpackung aus einer Folie besteht, die wasserlöslich ist. Dementsprechend kann das kosmetische Produkt dieser Ausführungsform in eine mit Wasser gefüllte Flasche gegeben werden, wobei die Folie sich auflöst und das Konzentrat freisetzt.

Unter dem Begriff "wasserlösliche Folie" wird erfindungsgemäß eine Folie verstanden, die bricht, sobald die Folie von Wasser umgeben ist.

Es ist bevorzugt, wenn der in den obigen Ausführungsformen beschriebene wasserlösliche Film Polyvinylalkohol, Gelantin und / oder Alginate umfasst oder daraus besteht, wobei Polyvinylalkohol bevorzugt ist.

In einer weiteren Ausführungsform liegt, das Konzentrat als Pulver in einer Verpackung vor, wobei die Verpackung biologisch abbaubar ist. Entsprechend umfasst die Verpackung bevorzugt kein Kunststoff.

Sofern das Konzentrat in eine Flasche mit Wasser gemischt vorliegt, wird erfindungsgemäß vorteilhaft die Reinigungszusammensetzung so angewendet, dass die Reinigungszusammensetzung mittels eines handbetriebenen Pumpkolben entnommen wird. Geeignete Pumpkolben erlauben dabei insbesondere vorteilhaft ein Aufschäumen der Reinigungszusammensetzung.

### Vergleichsversuche und Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Nachfolgende Tabellen zeigen verschiedene Konzentrat, sowie die Beobachtung.

Die Messung der Viskosität erfolgte wie in der obigen Beschreibung angegeben. Bsp.1 bis Bsp.4 sind Vergleichsbeispiele. Bsp. 5 bis Bsp.8 sind erfindungsgemäß.

| **Inhaltstoffe** | **Bsp.1** | **Bsp.2** | **Vgl.1** | **Vgl.2** | **Vgl.3** |
|---|---|---|---|---|---|
| Citric Acid | 15,63 | 15,63 | 15,63 | 15,63 | 15,63 |
| Sodium Benzoate | 15,63 | 15,63 | 15,63 | 15,63 | 15,63 |
| 99% Sodium Citrate + 1% Aqua | 2,34 | 2,34 | 2,34 | 2,34 | 2,34 |
| Magnesium Carbonate Hydroxide | 2,34 | 2,34 | 2,34 | 2,34 | 2,34 |
| 95% Disodium Lauryl Sulfosuccinate + 5% Aqua | 15,63 | 15,63 | 15,63 | 15,63 | 15,63 |
| Maltodextrin | 7,81 | 7,81 | 7,81 | 7,81 | 7,81 |
| Hydroxyacetophenone | 5,47 | 5,47 | 5,47 | 5,47 | 5,47 |
| 97% Sodium Coco Sulfate + 3% Aqua | 19,52 | 19,52 | 19,52 | 19,52 | 19,52 |
| Caesalpinia Spinosa Gum | 15,63 | | | | |
| Cellulose Gum | | 15,63 | | | |
| Dehydroxanthan Gum | | | 15,63 | | |
| Hydroxypropyl Guar | | | | 15,63 | |
| Xanthan Gum | | | | | 15,63 |

Die vorstehend aufgeführten Konzentrate wurden mit Wasser im Gewichtsverhältnis 14,625:1 gemischt. Zum Mischen wurden diese 20 Sekunden geschüttelt. 24h später wurden die Lösungs- und Verdickungseigenschaften beurteilt:

| **Inhaltstoffe** | **Bsp.1** | **Bsp.2** | **Vgl.1** | **Vgl.2** | **Vgl.3** |
|---|---|---|---|---|---|
| Klumpen/ Ausfällungen | Keine Klümpchen | Keine Klümpchen | Dehydroxanthan Gum setzt sich ab, Phasentrennung | Ungelöst Puderklumpen | Ungelöste Puderklumpen |
| Verdickung | Gleichmäßig verdickt | Verdickt nur oben, Rest wasserdünn | wasserdünn | wasserdünn | wasserdünn |

Weitere erfindungsgemäße Beispiele sind nachfolgend dargestellt:

| **Inhaltstoff** | **Bsp. 3** | **Bsp. 4** | **Bsp. 5** | **Bsp. 6** | **Bsp. 7** | **Bsp.8** |
|---|---|---|---|---|---|---|
| Citric Acid | 23,87 | 25,64 | 16,65 | 20,80 | 19,67 | 21,65 |
| Sodium Benzoate | 20,06 | 15,54 | 18,34 | 17,33 | 17,95 | 15,64 |
| 99% Sodium Citrate + 1% Aqua | 3,80 | 1,30 | 4,86 | 2,60 | 3,97 | 3,64 |
| Magnesium Carbonate Hydroxide | 3,55 | 8,30 | 6,63 | 5,20 | 4,86 | 4,80 |
| 95% Disodium Lauryl Sulfosuccinate + 5% Aqua | 16,05 | 13,64 | 17,88 | 17,33 | 10,67 | 20,67 |
| Maltodextrin | 3,85 | 1,66 | 5,34 | 3,47 | 6,84 | 4,09 |
| Hydroxyacetophenone | 5,93 | 2,82 | 4,37 | 6,07 | 6,90 | 7,49 |
| 97% Sodium Coco Sulfate + 3% Aqua | 15,88 | 22,34 | 20,46 | 21,65 | 23,59 | 17,37 |
| Caesalpinia Spinosa Gum | 1,00 | 0,76 | 0,15 | 0,35 | 0,65 | 0,70 |
| Fragrance | 5,90 | 6,20 | 4,55 | 5,20 | 4,90 | 3,95 |
| | | | | | | |
| Aqua | ad 100 | ad 100 | ad 100 | ad 100 | ad 100 | ad 100 |

## Patentansprüche

1. Kosmetische Reinigungskonzentrat in fester Form aufweisend, bezogen auf das Gesamtgewicht des Konzentrats,
a) ein oder mehrere unter Normalbedingungen feste Tenside und
b) Caesalpinia Spinosa Gum und/oder Cellulose Gum;
**dadurch gekennzeichnet, dass** das Konzentrat weniger als 7 Gew.-% unter
Normalbedingungen flüssige Inhaltstoffe enthält und Sodium Benzoate enthalten ist.

2. Konzentrat nach Anspruch 1 **dadurch gekennzeichnet, dass** die festen Tenside gewählt sind aus der Gruppe Sodium Myristoyl Glutamate, Sodium Lauroyl Glutamate, Sodium Stearoyl Glutamate, Sodium Cocoyl Glutamate, Sodium Coco Sulfate, Disodium Lauryl Sulfosuccinate und Sodium Methyl Cocoyl Taurate.

3. Konzentrat nach einem der vorhergehenden Ansprüche **dadurch gekennzeichnet, dass** Disodium Lauryl Sulfosuccinate und/oder Sodium Coco Sulfate im Reinigungskonzentrat enthalten sind.

4. Konzentrat nach einem der vorhergehenden Ansprüche **dadurch gekennzeichnet, dass** der Anteil von unter Normalbedingungen festen Tenside von 0,1 bis 50 Gew.-%, bevorzugt von 15 bis 45 Gew.-% und insbesondere bevorzugt von 25 bis 44 Gew.-%, bezogen auf das Gesamtgewicht des Reinigungskonzentrats, beträgt.

5. Konzentrat nach einem der vorhergehenden Ansprüche **dadurch gekennzeichnet, dass** Disodium Lauryl Sulfosuccinate in einem Anteil von 8 bis 30 Gew.-%, bevorzugt 10 bis 25 Gew.-% und insbesondere bevorzugt von 12 bis 20 Gew.-%, bezogen auf das Gesamtgewicht des Reinigungskonzentrats, enthalten ist.

6. Konzentrat nach einem der vorhergehenden Ansprüche **dadurch gekennzeichnet, dass** Sodium Coco Sulfate in einem Anteil von 12 bis 34 Gew.-%, bevorzugt 14 bis 30 Gew.-% und insbesondere bevorzugt von 18 bis 25 Gew.-%, bezogen auf das Gesamtgewicht des Reinigungskonzentrats, enthalten ist.

7. Konzentrat nach einem der vorhergehenden Ansprüche **dadurch gekennzeichnet, dass** Disodium Lauryl Sulfosuccinate und Sodium Coco Sulfate enthalten sind und weiterhin das Gewichtsverhältnis von Disodium Lauryl Sulfosuccinate zu Sodium Coco Sulfate von 1,5:1 bis 1:2, bevorzugt 1:1 bis 1:1,5 und insbesondere bevorzugt 1:1,1 bis 1:1,4 beträgt.

8. Konzentrat nach einem der vorhergehenden Ansprüche **dadurch gekennzeichnet, dass** Gesamtanteil von Caesalpinia Spinosa Gum und/oder Cellulose Gum von 0,1 bis 30 Gew.-%, bevorzugt von 0,2 bis 25 Gew.-%, weiter bevorzugt von 0,25 bis 20 Gew.-% und insbesondere bevorzugt 0,3 bis 20 Gew.-%, bezogen auf das Gesamtgewicht des Konzentrats, beträgt.

9. Konzentrat nach einem der vorhergehenden Ansprüche **dadurch gekennzeichnet, dass** das Konzentrat frei von Xanthan Gum, Sphingomonas Ferment Extract, Dehydroxanthan Gum und/oder Hydroxypropyl Guar ist.

10. Konzentrat nach einem der vorhergehenden Ansprüche **dadurch gekennzeichnet, dass** das Konzentrat Maltodextin umfasst, wobei es vorteilhaft ist, wenn der Anteil von Maltodextrin von 0,1 bis 20 Gew.-%, bevorzugt von 2 bis 14 Gew.-% und insbesondere bevorzugt von 3 bis 9 Gew.-%, bezogen auf das Gesamtgewicht des Konzentrats, beträgt.

11. Konzentrat nach einem der vorhergehenden Ansprüche **dadurch gekennzeichnet, dass** Sodium Benzoate enthalten ist, wobei der Anteil von Sodium Benzoate von 0,1 bis 30 Gew.-%, bevorzugt von 5 bis 25 Gew.-% und insbesondere bevorzugt von 10 bis 20 Gew.-%, bezogen auf das Gesamtgewicht des Konzentrats, beträgt.

12. Konzentrat nach einem der vorhergehenden Ansprüche **dadurch gekennzeichnet, dass** der Anteil der unter Normalbedingungen flüssigen Zusatzstoffe weniger als 6,85 Gew.-% und insbesondere weniger als 6,5 Gew.-%, bezogen auf das Gesamtgewicht des Konzentrats, beträgt.

13. Konzentrat nach einem der vorhergehenden Ansprüche **dadurch gekennzeichnet, dass** der Anteil von Wasser bevorzugt weniger als 5 Gew.-% und insbesondere bevorzugt weniger als 2 Gew.-%, bezogen auf das Gesamtgewicht des Konzentrats, beträgt.

14. Konzentrat nach einem der vorhergehenden Ansprüche **dadurch gekennzeichnet, dass** das Konzentrat in Tablettenform oder Pulverförmig vorliegt.

15. Kosmetisches Produkt aufweisend eine Verpackung und ein Konzentrat gemäß einem der vorstehenden Ansprüche, **dadurch gekennzeichnet, dass** die Verpackung das erfindungsgemäße Konzentrat umschließt.

16. Kosmetisches Produkt nach Anspruch 15 **dadurch gekennzeichnet, dass** die Verpackung des Konzentrats mindestens einen Abschnitt aufweist, der aus einer wasserlöslichen Folie besteht.

## Claims

1. Cosmetic cleansing concentrate in solid form comprising, based on the total weight of the concentrate,
a) one or more surfactants that are solid under standard conditions and
b) Caesalpinia Spinosa Gum and/or Cellulose Gum; **characterized in that** the concentrate comprises less than 7% by weight of ingredients that are liquid under standard conditions, and Sodium Benzoate is present.

2. Concentrate according to Claim 1, **characterized in that** the solid surfactants are selected from the group of Sodium Myristoyl Glutamate, Sodium Lauroyl Glutamate, Sodium Stearoyl Glutamate, Sodium Cocoyl Glutamate, Sodium Coco Sulfate, Disodium Lauryl Sulfosuccinate and Sodium Methyl Cocoyl Taurate.

3. Concentrate according to either of the preceding claims, **characterized in that** Disodium Lauryl Sulfosuccinate and/or Sodium Coco Sulfate are present in the cleansing concentrate.

4. Concentrate according to any of the preceding claims, **characterized in that** the proportion of surfactants that are solid under standard conditions is from 0.1 to 50% by weight, preferably from 15 to 45% by weight and particularly preferably from 25 to 44% by weight, based on the total weight of the cleansing concentrate.

5. Concentrate according to any of the preceding claims, **characterized in that** Disodium Lauryl Sulfosuccinate is present at a proportion of 8 to 30% by weight, preferably 10 to 25% by weight and particularly preferably from 12 to 20% by weight, based on the total weight of the cleansing concentrate.

6. Concentrate according to any of the preceding claims, **characterized in that** Sodium Coco Sulfate is present at a proportion of 12 to 34% by weight, preferably 14 to 30% by weight and particularly preferably from 18 to 25% by weight, based on the total weight of the cleansing concentrate.

7. Concentrate according to any of the preceding claims, **characterized in that** Disodium Lauryl Sulfosuccinate and Sodium Coco Sulfate are present and furthermore the ratio by weight of Disodium Lauryl Sulfosuccinate to Sodium Coco Sulfate is from 1.5:1 to 1:2, preferably 1:1 to 1:1.5 and particularly preferably 1:1.1 to 1:1.4.

8. Concentrate according to any of the preceding claims, **characterized in that** the total proportion of Caesalpinia Spinosa Gum and/or Cellulose Gum is from 0.1 to 30% by weight, preferably from 0.2 to 25% by weight, more preferably from 0.25 to 20% by weight and particularly preferably from 0.3 to 20% by weight, based on the total weight of the concentrate.

9. Concentrate according to any of the preceding claims, **characterized in that** the concentrate is free from Xanthan Gum, Sphingomonas Ferment Extract, Dehydroxanthan Gum and/or Hydroxypropyl Guar.

10. Concentrate according to any of the preceding claims, **characterized in that** the concentrate comprises Maltodextrin, wherein it is advantageous if the proportion of Maltodextrin is from 0.1 to 20% by weight, preferably from 2 to 14% by weight and particularly preferably from 3 to 9% by weight, based on the total weight of the concentrate.

11. Concentrate according to any of the preceding claims, **characterized in that** Sodium Benzoate is present, wherein the proportion of Sodium Benzoate is from 0.1 to 30% by weight, preferably from 5 to 25% by weight and particularly preferably from 10 to 20% by weight, based on the total weight of the concentrate.

12. Concentrate according to any of the preceding claims, **characterized in that** the proportion of additives that are liquid under standard conditions is less than 6.85% by weight and especially less than 6.5% by weight, based on the total weight of the concentrate.

13. Concentrate according to any of the preceding claims, **characterized in that** the proportion of water is preferably less than 5% by weight and particularly preferably less than 2% by weight, based on the total weight of the concentrate.

14. Concentrate according to any of the preceding claims, **characterized in that** the concentrate is in tablet form or powder form.

15. Cosmetic product comprising a packaging and a concentrate according to any of the preceding claims, **characterized in that** the packaging encloses the concentrate according to the invention.

16. Cosmetic product according to Claim 15, **characterized in that** the packaging of the concentrate has at least one section consisting of a water-soluble film.

## Revendications

1. Concentré de nettoyage cosmétique sous forme solide, comprenant, par rapport au poids total du concentré,
a) un ou plusieurs tensioactifs solides dans des conditions normales et
b) de la Caesalpinia Spinosa Gum et/ou de la Cellulose Gum ; **caractérisé en ce que** le concentré contient moins de 7 % en poids de composants liquides dans des conditions normales et du benzoate de sodium est contenu.

2. Concentré selon la revendication 1, **caractérisé en ce que** les tensioactifs solides sont choisis dans le groupe du Sodium Myristoyl Glutamate, Sodium Lauroyl Glutamate, Sodium Stearoyl Glutamate, Sodium Cocoyl Glutamate, Sodium Coco Sulfate, Disodium Lauryl Sulfosuccinate et Sodium Methyl Cocoyl Taurate.

3. Concentré selon l'une quelconque des revendications précédentes, **caractérisé en ce que** du Disodium Lauryl Sulfosuccinate et/ou du Sodium Coco Sulfate sont contenus dans le concentré de nettoyage.

4. Concentré selon l'une quelconque des revendications précédentes, **caractérisé en ce que** la teneur en tensioactifs solides dans des conditions normales est de 0,1 à 50 % en poids, de préférence de 15 à 45 % en poids et de manière particulièrement préférée de 25 à 44 % en poids, par rapport au poids total du concentré de nettoyage.

5. Concentré selon l'une quelconque des revendications précédentes, **caractérisé en ce que** du Disodium Lauryl Sulfosuccinate est contenu en une proportion de 8 à 30 % en poids, de préférence de 10 à 25 % en poids et de manière particulièrement préférée de 12 à 20 % en poids, par rapport au poids total du concentré de nettoyage.

6. Concentré selon l'une quelconque des revendications précédentes, **caractérisé en ce que** du Sodium Coco Sulfate est contenu en une proportion de 12 à 34 % en poids, de préférence de 14 à 30 % en poids et de manière particulièrement préférée de 18 à 25 % en poids, par rapport au poids total du concentré de nettoyage.

7. Concentré selon l'une quelconque des revendications précédentes, **caractérisé en ce que** du Disodium Lauryl Sulfosuccinate et du Sodium Coco Sulfate sont contenus et **en ce que** le rapport en poids du Disodium Lauryl Sulfosuccinate au Sodium Coco Sulfate est de 1,5:1 à 1:2, de préférence de 1:1 à 1:1,5 et de manière particulièrement préférée de 1:1,1 à 1:1,4.

8. Concentré selon l'une quelconque des revendications précédentes, **caractérisé en ce que** la teneur totale en Caesalpinia Spinosa Gum et/ou Cellulose Gum est de 0,1 à 30 % en poids, de préférence de 0,2 à 25 % en poids, de préférence supplémentaire de 0,25 à 20 % en poids et de manière particulièrement préférée de 0,3 à 20 % en poids, par rapport au poids total du concentré.

9. Concentré selon l'une quelconque des revendications précédentes, **caractérisé en ce que** le concentré est exempt de Xanthan Gum, de Sphingomonas Ferment Extract, de Dehydroxanthan Gum et/ou de Hydroxypropyl Guar.

10. Concentré selon l'une quelconque des revendications précédentes, **caractérisé en ce que** le concentré comprend de la maltodextrine, il étant avantageux que la teneur en maltodextrine soit de 0,1 à 20 % en poids, de préférence de 2 à 14 % en poids et de manière particulièrement préférée de 3 à 9 % en poids, par rapport au poids total du concentré.

11. Concentré selon l'une quelconque des revendications précédentes, **caractérisé en ce que** du Sodium Benzoate est contenu, la teneur en Sodium Benzoate étant de 0,1 à 30 % en poids, de préférence de 5 à 25 % en poids et de manière particulièrement préférée de 10 à 20 % en poids, par rapport au poids total du concentré.

12. Concentré selon l'une quelconque des revendications précédentes, **caractérisé en ce que** la teneur en additifs liquides dans des conditions normales est inférieure à 6,85 % en poids et en particulier inférieure à 6,5 % en poids, par rapport au poids total du concentré.

13. Concentré selon l'une quelconque des revendications précédentes, **caractérisé en ce que** la proportion d'eau est de préférence inférieure à 5 % en poids et de manière particulièrement préférée inférieure à 2 % en poids, par rapport au poids total du concentré.

14. Concentré selon l'une quelconque des revendications précédentes, **caractérisé en ce que** le concentré se présente sous forme de comprimé ou sous forme de poudre.

15. Produit cosmétique comprenant un emballage et un concentré selon l'une quelconque des revendications précédentes, **caractérisé en ce que** l'emballage entoure le concentré selon l'invention.

16. Produit cosmétique selon la revendication 15, **caractérisé en ce que** l'emballage du concentré comprend au moins une section constituée d'un film hydrosoluble.
